(19) Europäisches Patentamt
European Patent Office
Office européen des brevets

(11) Veröffentlichungsnummer: **0 068 250**
**B1**

(12) # EUROPÄISCHE PATENTSCHRIFT

(45) Veröffentlichungstag der Patentschrift: **26.02.86**

(21) Anmeldenummer: **82105150.5**

(22) Anmeldetag: **12.06.82**

(51) Int. Cl.4: **C 07 C 65/24,** C 07 C 65/40, C 07 C 59/68, C 07 C 59/90, C 07 C 69/76, C 07 C 69/618, C 07 C 51/367, C 07 C 67/29, A 61 K 31/19, A 61 K 31/235

(54) **Dioxybenzoletherderivate, diese enthaltende Arzneimittel, Verfahren zu ihrer Herstellung und ihre Verwendung.**

(30) Priorität: **26.06.81 DE 3125059**

(43) Veröffentlichungstag der Anmeldung: **05.01.83 Patentblatt 83/1**

(45) Bekanntmachung des Hinweises auf die Patenterteilung: **26.02.86 Patentblatt 86/9**

(84) Benannte Vertragsstaaten: **AT BE CH DE FR GB IT LI NL SE**

(56) Entgegenhaltungen:
**AU - A - 477 982**
**DE - A - 1 518 011**
**DE - A - 1 518 020**
**DE - A - 1 668 938**
**DE - A - 2 000 299**
**DE - A - 2 307 038**
**DE - A - 2 521 915**
**DE - A - 2 716 189**
**DE - A - 2 802 281**
**GB - A - 1 262 078**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Böshagen, Horst, Dr., Wiesenstrasse 4, D-5657 Haan (DE)**
Erfinder: **Hörlein, Ulrich, Dr., Steinmetzstrasse 17, D-5600 Wuppertal 11 (DE)**
Erfinder: **Reinhardt, Gerd, Dr., Am Eckbusch 55a, D-5600 Wuppertal 1 (DE)**
Erfinder: **Seuter, Friedel, Dr., Moospfad 16, D-5600 Wuppertal 1 (DE)**
Erfinder: **Perzborn, Elisabeth, Dr., Am Tescherbusch 13, D-5600 Wuppertal 11 (DE)**

**EP 0 068 250 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die vorliegende Erfindung betrifft neue Dioxybenzoletherderivate, ein Verfahren zu ihrer Herstellung, Arzneimittel enthaltend diese Verbindungen sowie ihre Verwendung zur Herstellung von Arzneimitteln, insbesondere von Thromboxan-Synthetasehemmern, sowie von Antiatherosklerotika.

Dioxnybenzolether und Verfahren zu ihrer Herstellung sind bereits bekannt (vgl. z.B Beilst. *6, 771, 772, 814, 815, 843—846, Beilst. 6,* I. Erg. Wk. 383, 384, 402, 416, Beilst. *6,* II. Erg. Wk. 779—783, 813—816, 839 ff, Beilst. *6,* III. Erg. Wk. 4205—4227, 4305—4319, 4385—4413, Beilst. *6,* IV. Erg. Wk. 5564—5581, 5663—5672). Eine antithrombotische und antiatherosklerotische Wirkung dieser Stoffklasse ist bisher noch nicht beschrieben worden. Einige Dioxybenzyletherderivate sind auf Coccidiose-Wirkung geprüft worden, wobei keine befriedigenden Wirkungen gefunden wurden (vgl. J.med.Chem. *21,* 357 (1987)).

Die vorliegende Erfindung betrifft neue Dioxybenzoletherderivate der allgemeinen Formel (I)

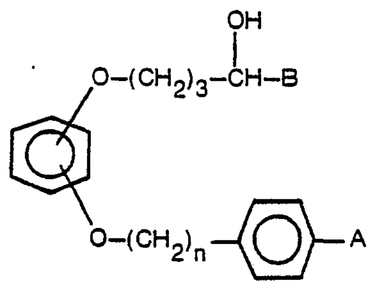

in welcher die beiden Ethergruppen in ortho- oder meta-Stellung zueinander stehen,

A eine der Grupen
$—COOH$,
$—COOC_2H_5$,
$—CH=CH—COOH$ oder
$—CH=CH—COOC_2H_5$,

B einen geradkettigen Alkylrest mit 3—6 C-Atomen und

n 1—3

bedeuten, sowie deren Additionssalze.

Die Verbindungen der allgemeinen Formel werden erfindungsgemäß hergestellt, indem man Hydroxybenzolderivate der allgemeinen Formel

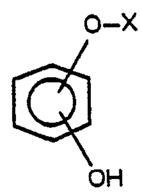

in welcher

X für eine Schutzgruppe steht,

in inerten organischen aprotischen Lösungsmitteln in Gegewart der äquivalenten Menge einer starken Base mit Verbindungen der allgemeinen Formel

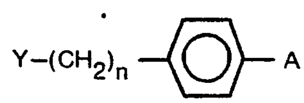

wobei

n und A die oben angegebene Bedeutung haben und

Y eine austretende Gruppe darstellt,

bei Temperaturen zwischen 0 und 150°C umsetzt, anschließend die Schutzgruppe X nach üblichen Methoden abspaltet und dann mit Verbindungen der allgemeinen Formel

$$Y'—(CH_2)_3—\underset{}{\overset{OH}{\overset{|}{CH}}}—B$$

wobei

B die oben angegebene Bedeutung hat und

Y' die Bedeutung von Y besitzt, ohne mit dieser identisch sein zu müssen,

in der oben beschreibenen Weise zu Verbindungen der allgemeinen Formel (I) verethert.

Als Schutzgruppen des Subsatituenten X seien vorzugsweise genannant Acyl mit 1 bis 4 Kohlenstoffatomen, insbesondere Acetyl, Benzoyl, Benzyl, Tetrahydropyranyl und Trialkylsilyl mit 1 bis 4 Kohlenstoffatomen in den Alkylresten.

Für den Substituenten Y seien als austretende Gruppen vorzugsweise genannt Halogen, insbesondere Chlor und Brom, Tolyl und Alkylsulfonyl mit 1 bis 4 Kohlenstoffatomen im Alkylrest.

Für die als Säurefänger eingesetzten Basen im Reaktionsmedium seien vorzugsweise genannt Alkalihydroxide, Alkalihydride, Alkaliamide, Alkalialkoholate und Butyllithium, wobei als Alkalimetalle insbesondere Natrium und Kalium geeignet sind.

Als inerte organische aprotische Lösungsmittel seien vorzugsweise gennant Toluol, Benzol, Dimethylformamid und Dimethylsulfoxid sowie Gemische dieser Lösungsmittel.

Die Reaktion kann bei unterschiedlichen Drucken durchgeführt werden, vorzugsweise arbeitet man bei Normaldruck. Die Reaktion wird bei Temperaturen zwischen 0 und 150°C, insbesondere zwischen 50 und 120°C, durchgeführt.

Die erfindungsgemäßen Verbindungen können eingesetzt werden, wenn eine Blutplättchen-Aggregation verhindert, die klebrigen Eigenschaften von Plättchen vermindert und damit die Bildung von Blutpfropfen (Thromben) verhindert werden sol (Thromboseprophylaxe). Die bei den erfindugsgemäßen Verbindungen gefundene Thrombinhemmung kann ebenso die Entstehung von Thromben hemmen bzw. verhindern (Thromboseprophylaxe). Eine zusätzliche Eigenschaft der erfindungsgemäßen Verbindungen ist die Hemmung der Thromboxansynthese, womit die Entstehung des die Thrombozytenaggregation auslöenden und gefäßkonstringierend wirkenden Thromboxan ($TXA_2$) unterbunden wird. Die Verbindungen hemmen in experimentellen Atherosklerose-Modellen auch die durch Verletzung und Hyperlipidämie ausgelöste Proliferation der Gefäß-Intima, die als ein Frühstadium atherosklerotischer Veränderungen angesehen wird.

Die folgenden Testmethoden zeigen die überraschende Wirkung der erfindungsgemäßen Verbindungen:

1. Bestimmung der antiarteriosklerotischen Wirkung

Bei Ratten wird ein Abschnitt der Arteriacarotis operativ freigelegt und auf −10°C unterkühlt. Die Tiere werden nach der Operation für 10 Tage mit einer cholestinreichen Diät gefüttert. In dem geschädigten Gefäß bilden sich dabei atherosklerotische Ablagerungen, deren Größe histologisch oder durch Wägung ermittelt wird. (Vgl. F. Seuter et. al., "Experimentally induced Thromboatherosclerosis in rats and rabbits", Folia Angiologica *28*, 85—87 (1980)).

Es zeigt sich daß z.B. bei oraler Verabreichung von einer Dosis von 100 mg/kg der Verbindung des Beispiels 8 eine Hemmung der Ablagerung um 59 % bewirkt wird.

2. Hemmung des Thrombins

Die zu untersuchenden Substanzen werden in einer Dosis von 1 bis 50 µl in ein Teströhrchen vorgelegt und mit Puffer aufgefüllt. Dann werden die Ansätze mit 20 µl einer Thrombin-Lösung versetzt und 20 min. bei Raumteperatur stehen gelassen. Anschließend wird die Reaktion durch eine Substratlösung gestartet. Als Maß der Aktivität wird der Extinktionsanstieg pro Minute bestimmt.

Bei einer Dosis von 10—20 µg/ml zeigen zahlreiche erfindungsgemäße Verbindungen eine Hemmung. Die Verbindung des Beispiels 19 zeigt in einer Gesamtkonzentration von $1 \times 10^{-5}$ g/l eine Thrombinhemmung von 50 %.

3. Thromboxansynthesehemmung

Einige der erfindungsgemäßen Verbindungen besitzen eine sehr spezifische inhibierende Wirkung auf die Thromboxansynthethase, ohne die Cyclooxigenase stärker zu beeinflußen. Die Verbindung des Beispiels 6 zeigt bereits bei einer Konzentration von $3 \times 10^{-5}$ g/ml eine Hemmung auf die Thromboxansynthease um 86 &. Bei der gleichen Dosis zeigt die Verbindung des Beispiels 10 eine Hemmung um 96 %. Die Untersuchungen erfolgten analog zu bekannten Testmethoden (vgl. R. F. Furchgott et. al., Phamakol. Exp. Ther. *108*, 129—143 (1953) und J. M. Bailey et. al., Prostaglandins *13*, 479-492 (1977)).

Aufgrund der vorgenannten Wirkqualitäten eignen sich die erfindungsgemäßen Verbindungen beispielsweise zur Behandlung oder Verhinderung von Herzinfarkten, der Angina pectoris, thromboembolischer Erkrankungen im venösen und arteriellen Bereich (postoperativ, transitorisch ischämische Attacken, Amaurosis fugax etc.), anderer vaskulärer Krankheitsbilder wie der Arterosklerose.

Aus DE—AS 15 18 011 sind bereits (4-Chlor-2-methyl-phenoxy)-(4-chlorphenyl)essigsäuren bekannt geworden, die den Cholesterinspiegel des Blutes zu senken vermögen. Die DE—OS 27 16 188 beschreibt Phenoxypropionsäurederivate mit hypolipodämischer, hypocholesterinämischer oder hypotriglyceridämischer Wirkung.

Arteriosklerose ist aber eine multifaktorielle Erkrankung, die bei Tieren durch eine Vielzahl von Induktoren ausgelöst werden kann, z.B. durch Hypercholesterinämie. Plaques entstehen dabei jedoch erst nach mehrmonatiger Behandlung. Unter diesen Versuchsbedingungen sind Hypolipidämika wirksam. Der Wirkungsmechanismus besteht in der Hemmung des atherogenen Stimulus.

In den vorstehenden Versuchen hingegen wird eine Atherosklerose durch eine traumatische Verletzung der Gefäßwand erzeugt. Die Folgeerscheinung, Intimaproliferationen und Atherosklerose, treten mit und ohne Cholesterinfütterung der Tiere ein. Es handelt sich um ein Kurzzeitmodell, die

Intimaproliferationen entwickeln sich innerhalb von 10 Tagen. Die diesen Vorgängen zugrunde liegenden Basismechanismen sind bisher weitgehend unbekannt.

Die positiven Effekte der erfindungsgemäßen Substanzen waren nicht vorhersehbar, denn hypolipidämische Substanzen, z.B. Clofibrat oder Cholestyramin, sind absolut unwirksam in diesem Modell; Effekte wurden bisher nur mit Dexamethason oder parenteralter Heparingabe beobachtet.

Die erfindungsgemäßen Verbindungen eignen sich darüberhinaus zur Behandlung von thromboembolischen Erkrankungen. Dies beruht auf der völlig überraschenderweise gefundenen Thromboxansynthesehemmung.

Die Thromboxansynthetase ist ein Emzym, das bei der Arachidonisäuremtabolisierung über den Cyclooxygenaseweg die Umwandlung von Endoperoxiden zu Thromboxan katalysiert.

Thromboxan, $TXA_2$ ist ein sehr potenter Vasokonstriktor und Auslöser der Thrombozytenaggregation, wordurch eine Thrombusbildung im arteriellen System ausgelöst wird.

Eine Querverbindung zum Lipidstoffwechsel besteht nicht. Die biologischen Eigenschaften der erfindungsgemäßen Verbindungen sind also in hohem Maße überraschend.

Die neuen Wirkstoffe können in bekannter Weise in die üblichen Formulierungen übergeführt werden, wie Tabletten, Kapseln, Dragees, Pillen, Granulate, Aerosole, Sirupe, Emulsionen, Suspensionen und Lösungen, unter Verwendung inerter, nicht-toxischer pharmazeutisch geeigneter Trägerstoffe oder Lösungsmittel. Hierbei soll die therapeutisch wirksame Verbindung jeweils in einer Konzentration von etwa 0,5 bis 90 Gew.−% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielhaft aufgeführt:

Wasser, nicht-toxische organische Lösungsmittel, wie Paraffine (z.b. Erdölfraktionen), pflanzliche Öle (z.B. erdnuß-/Sesamöl), Alkohole (z.B. Ethylalkohol, Glycerin), Glykole (z.B. Propylenglykol, Polyethylenglykol), feste Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Roh-, Milch- und Traubenzucker), Emulgiermittel, wie nicht-ionogene anionische Emulgatoren (z.B. Polyoxyethylen-Fettsäure-Ester, Polyoxyethylen-Fettalkohol-Ether, Alkylsulfonate und Arylsulfonate), Dispergiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich außer den gennanten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspension und/oder Elixieren, die für orale Anwendungen gedacht sind, können die Wirkstoffe außer mit den o.g. Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermatrialien eingesetzt werden.

Im allgemeinen hat es sich als vorteilhaft erwiesen, bei intravenöser Applikation Mengen von etwa 0,01 bis 10 mg/kg, vorzugsweise etwa 0,05 bis 5 mg/kg, Körpergewicht pro Tag zur Erzielung wirksamer Ergebnisse zu verabreichen, und bei oraler Applikation beträgt die Dosierung etwa 0,05 bis 20 mg/kg, vorzugsweise 0,5 bis 5 mg/kg Körpergewicht pro Tag.

Trotzdem kann es gegebenenfalls erforderlich sein, von den genannten Mengen abzuweichen, und zwar in Abhängigkeit vom Körpergewicht des Versuchstieres bzw. der Art des Applikationsweges, aber auch aufgrund der Tierart und deren individuellem Verhalten gegenüber dem Medikament bzw. der Art von dessen Formulierung und dem Zeitpunkt bzw. Intervall zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Megen kann es empfehlenswert sein, diese in mehrere Einzelgaben über den Tag zu verteilen. Für die Applikation in der Humanmedizin ist der gleiche Dosierungsspielraum vorgesehen. Sinngemäß gelten hierbei auch die obigen Ausführungen.

## Ausführungsbeispiele

### Beispiel 1

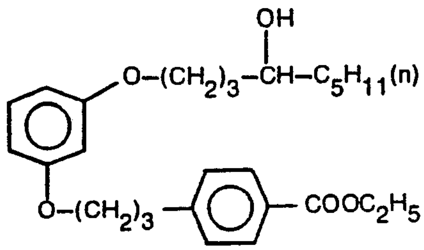

a) In eine Suspension von 3,33 g NaH (80 % Paraffin) in 180 ml eines über Molekularsieb getrockneten Benzol/DMF-Gemisches 1:1 tropft man eine Lösung von 20,2 g technischem Euresol (O-Monoacetylresorcin) in 40 ml des genannten Lösungsmittelgemisches ein. Man setzt eine Spur NaJ zu und erhitzt zum Sieden. Nun tropft man 29,5 g 4-Acetoxy-1-nonylchlorid zu und läßt 15 Stunden kochen. Nach dem Abkühlen wird abgesaugt, das Filtrat im Vakuum eingeengt, der Rückstand in Ether aufgenommen, die ehterische Lösung mit Wasser gewaschen und über $Na_2SO_4$ getrocknet. Nach dem Filtrieren wird der Eindampfrückstand destilliert. Ausbeute: 23 g Euresol-(4-acetoxy-nonyl)-ether vom $Kp_{0,1}$ 195-200°C.

b) 22 g Euresol-(4-acetoxy-nonyl)-ether wurden in einem Gemisch aus 35 ml 33 %iger Natronlauge, 65 ml Wasser und 200 ml Ethanol durch mehrstündiges Kochen verseift. Nach dem Abkühlen wird die Lösung im Vakuum eingedampft, der Rückstand in Wasser aufgenommen, die Lösung mit Kohle/Tonsil abgesaugt und das Filtrat mit Salzsäure sauer gestellt. Den abgeschiedenen (4-Hydroxy-nonyl)-resorcylether nimmt man in Ether auf, wäscht die etherische Lösung mit Wasser, trocknet über $Na_2SO_4$ und dampft nach dem Filtrieren im Vakuum vollständig ein. Der Rückstand wiegt 16,5 g.

c) In eine Suspension von 2,4 g NaH (80 % in Paraffin) in 120 ml eines wie oben behandelten Benzol/DMF-Gemisches 1:1 tropft man eine Lösung von 16 g (4-Hydroxy-nonyl)-resorcylether in 40 ml des genannten Lösungsmittelgemisches ein. Man setzt eine Spur NaJ zu und erhitzt zum Sieden. Dann tropft man 17,45 g 4-(3-Brompropyl)- benzoesäureethylester zu und läßt 15 Stunden kochen. Nach dem Erkalten saugt man ab, engt das Filtrat im Vakuum vollständig ein, verteilt den Rückstand zwischen verdünnter Natronlauge und Ether und dampft die über $Na_2SO_4$ getrocknete etherische Lösung ein. Die Destillation des Rückstandes im Retortenkolben mit weiten Querschnitten ergibt 22 g des Esters der oben verzeichneten Formel vom $Kp_{0,01}$ 262-267°C.

### Beispiel 2

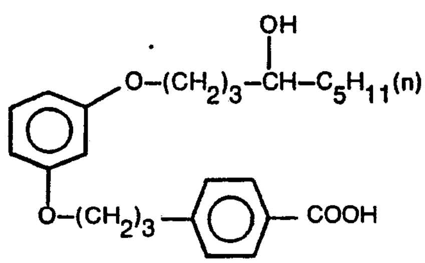

17,7 g Ester des Beispiels 1 werden mit 15 g NaOH in einem Gemisch aus 60 ml Wasser und 150 ml Ethanol einige Stunden gekocht. Nach dem Abkühlen wird im Vakuum eingedampft, der Rückstand in Wasser aufgenommen und die entstandene Lösung mit Kohle/Tonsil abgesaugt. Durch Ansäuern mit Salzsäure erhält man die Säure der oben verzeichneten Formel, die aus Ether/Petrolether umkristallisiert, bei 87°C schmilzt. Ausbeute: 12,7 g.

### Beispiel 3

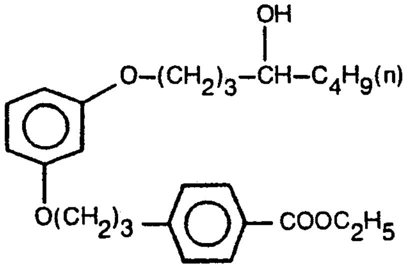

Wenn man Euresol analog zu Beispiel 1 mit 4-Acetoxy-1-octylchlorid umsetzt, die Verseifung der

Acetylgruppen wie beschrieben durchführt und den isolierten Resorcylether mit 4-(3-Brompropyl)-benzoesäureethylester umsetzt, so erhält man in jeweils entsprechenden Ausbeuten den Ester der oben verzeichneten Formel vom $Kp_{0,01}$ 258-260°C (Retortenkolben mit weiten Querschnitten).

Beispiel 4

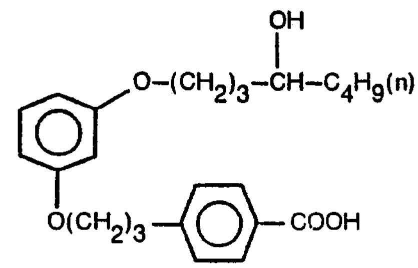

17,1 g Ester des Beispiels 3 werden mit 15 g NaOH in einem Gemisch aus 60 ml Wasser und 150 ml Ethanol einige Stunden gekocht. Die Aufarbeitung analog Beispiel 2 liefert die Säure der oben verzeichneten Formel vom Fp. 90-91°C (Ether/Petrolether). Ausbeute: 11,5 g.

Beispiel 5

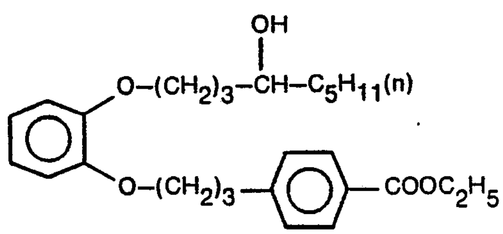

a) In eine Suspension von 6,7 g NaH (80 % in Paraffin) in 30 ml Benzol wird eine Lösung von 30,9 g technischem O-Monoacetylbrenzkatechin in 80 ml Benzol eingetropft, eine Spur NaJ hinzugegeben, 100 ml trockenes DMF zugesetzt und in der Siedehitze 50,5 g 4-Acetoxy-nonylchlorid eingetropft. Man läßt das Gemisch 15 Stunden kochen, saugt nach dem Abkühlen ab und dampft das Filtrat im Vakuum ein. Den Rückstand nimmt man in Ether auf, wäscht die etherische Lösung mit Natriumbicarbonatlösung in Wasser, trocknet sie ($Na_2SO_4$), dampft sie ein und destilliert den Eindampfrückstand. Ausbeute: 36,8 g O-Acetylbrenzkatechin-(4-acetoxy-nonyl)-ether vom $Kp_{0,01}$ 182-184°C.

b) 36 g O-Acetylbrenzkatechin-(4-acetoxy-nonyl)-ether werden in einer Lösung von 32,6 g NaOH in 200 ml Wasser und 30 ml Alkohol mehrere Stunden gekocht. Anschließend wird die Lösung im Vakuum eingedampft, der Rückstand zwischen Ether und Wasser verteilt, die etherische Lösung mit Wasser gewaschen, getrocknet ($Na_2SO_4$) und nach dem Filtrieren im Vakuum vollständig eingedampft. Ausbeute an Brenzkatechin-4-hydroxy-nonylether 25,6 g.

c) 25 g Brenzkatechin-4-hydroxy-nonylether werden nach Beispiel 1 c) bei Gegenwart von 3,75 g NaH (80 % in Paraffin) mit 27,0 g 4-(3-Brompropyl)-benzoesäureethylester umgesetzt und entsprechend aufgearbeitet. Der Ester der oben verzeichneten Formel kristallisiert beim Verreiben mit Petrolether. Ausbeute: 41,3 g vom Fp. 53-54°C.

Beispiel 6

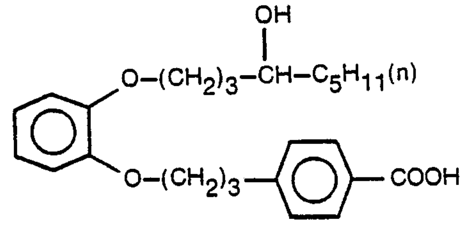

Wird der Ester des Beispiels 5 analog zu Beispiel 2 verseift, so erhält man die oben verzeichnete Säure vom Fp. 89-90°C (Ether/Petrolether).

Beispiel 7

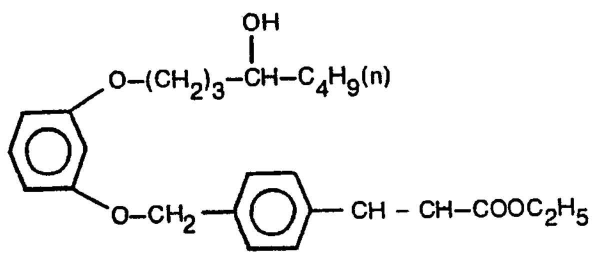

Wenn man (4-hydroxy-octyl)-resorcylether (Zwischenprodukt des Beispiels 3) mit (4- -Bromethyl)-(E)-zimtsäureethylester analog zu Beispiel 1 c) umsetzt, erhält man in 1 c) entpsrechender Ausbeute den oben verzeichneten Ester vom $Kp_{0,01}$ 228-230°C (Retortenkolben mit weiten Querschnitten).

Beispiel 8

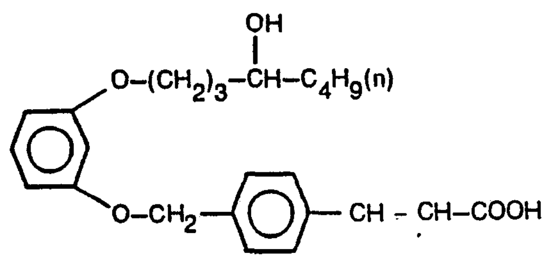

Wird der Ester des Beispiels 7 analog zu Beispiel 4 verseift, so erhält man die oben verzeichnete Säure vom Fp. 118-119°C (Cyclohexan).

Beispiel 9

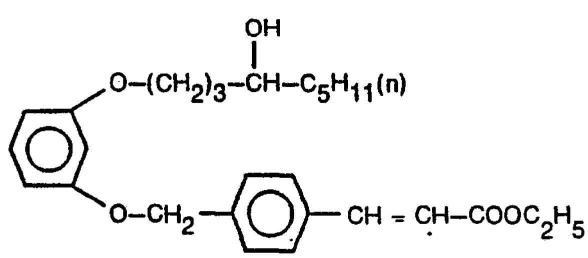

Wenn man (4-Hydroxy-nonyl)-resorcylether (Beispiel 1 b)) mit (4-α-Brommethyl)-(E)-zimtsäureethylester analog zu Beispiel 1 c) umsetzt, erhält man in 1 c) entsprechender Ausbeute den oben verzeichneten Ester vom $Kp_{0,01}$ 230-232°C (Retortenkolben mit weiten Querschnitten).

Beispiel 10

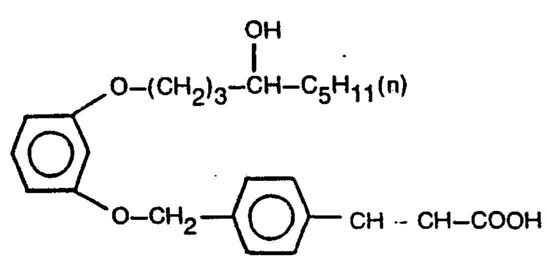

Wird der Ester des Beispiels 9 analog zu Beispiel 4 verseift (Kochzeit 30 Minuten), so erhält man die oben verzeichnete Säure in sehr guter Ausbeute. Fp. 115°C (Essigester/Ether).

Beispiel 11

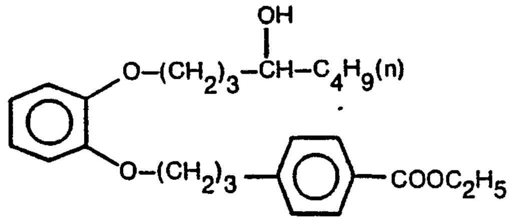

Wenn man O-Monoacetyl-brenzkatechin analog zu Beispiel 5 mit 4-Acetoxy-1-octylchlorid umsetzt, die Verseifung der Acetylgruppen wie beschrieben durchführt und den isolierten Brenzkatechinether mit 4-(3-Brompropyl)-benzoesäureethylester umsetzt, so erhält man in guten Ausbeuten den Ester der oben verzeichneten Formel vom $Kp_{0,01}$ 250-230°C.

Beispiel 12

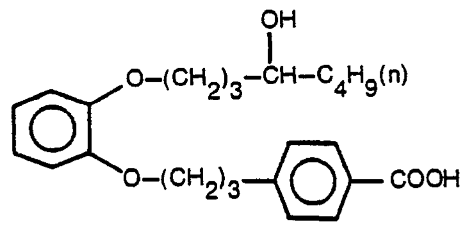

Wird der Ester des Beispiels 11 analog zu Beispiel 10 verseift, so erhält man die oben verzeichnete Säure in sehr guter Ausbeute. Fp. 69-70°C (Ether/Petrolether).

Beispiel 13

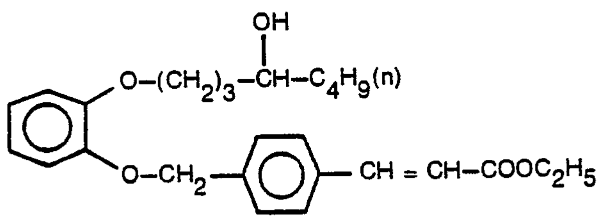

Wenn man Brenzkatechin-(4-hydroxy-octyl)-ether (Zwischenprodukt des Beispiels 11) mit (4-α-Brommethyl)-(E)-zimtsäureethylester analog zu Beispiel 5 umsetzt, erhält man in guter Ausbeute den oben verzeichneten Ester. Fp. 56-57°C (nach Verreiben mit Petrolether).

Beispiel 14

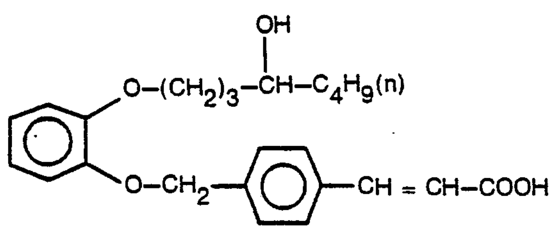

Wird der Ester des Beispiels 13 analog zu Beispiel 10 verseift, so erhält man in sehr guter Ausbeute die oben verzeichnete Säure. Fp. 110-111°C (wenig Ether/Petrolether).

Beispiel 15

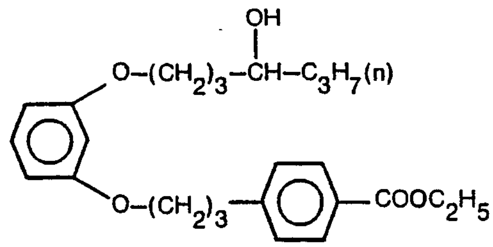

Wenn man Euresol analog zu Beispiel 1 mit 4-Acetoxy-heptylchlorid umsetzt, die Verseifung der

Acetylgruppen wie beschrieben durchführt und den isolierten Resorcylether mit 4-(3-Brompropyl)-benzoesäureethylester umsetzt, erhält man in jeweils entsprechenden Ausbeuten den Ester der oben verzeichneten Formel vom $Kp_{0,01}$ 240-245°C.

Beispiel 16

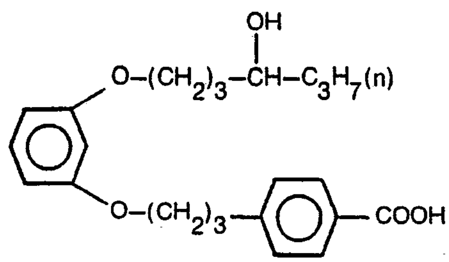

Wird der Ester des Beispiels 15 analog zu Beispiels 10 verseift, so erhält man in guter Ausbeute die oben verzeichnete Säure. Fp. 84-85°C (Toluol/Petrolether).

Beispiel 17

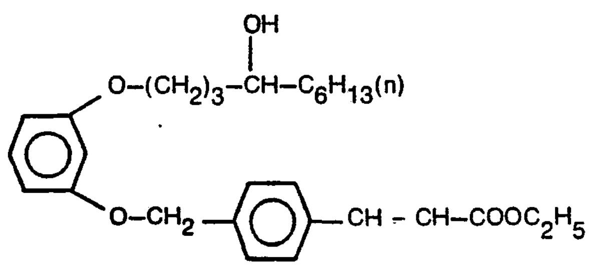

Wenn man Euresol analog zu Beispiel 1 mit 4-Acetoxydecylchlorid umsetzt, die Verseifung der Acetylgruppen wie beschrieben durchführt und den isolierten Resorcylether mit (4-α-Brommethyl)-(E)-zimtsäureethylester umsetzt, so erhält man in jeweils entsprechenden Ausbeuten den Ester der oben verzeichneten Formel vom $Kp_{0,01}$ 232-235°C, Fp. 49-50°C (Verreiben mit Petrolether).

Beispiel 18

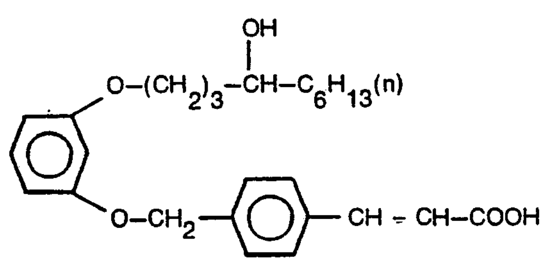

Wird der Ester des Beispiels 17 analog zu Beispiel 10 verseift, so erhält man in guter Ausbeute die oben verzeichnete Säure. Fp. 122-124°C (Essigsäuremethylester/Petrolether).

Beispiel 19

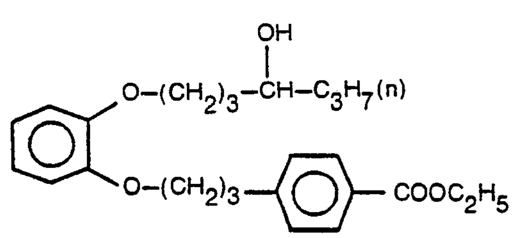

Wenn man O-Monoacetyl-brenzkatechin analog Beispiel 5 mit 4-Acetoxy-heptylchlorid umsetzt, die Verseifung der Acetylgruppen wie beschrieben durchführt und den isolierten Brenzkatechinether mit 4-(3-Brompropyl)-benzoesäureethylester umsetzt, so erhält man in guter Ausbeute den Ester der oben verzeichneten Formel. $Kp_{0,01}$ 224-227°C.

Beispiel 20

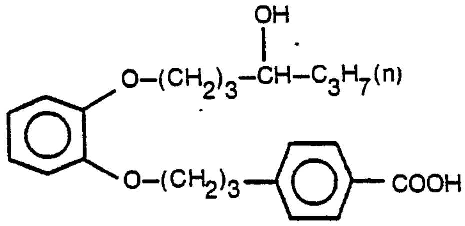

Wird der Ester des Beispiels 19 analog zu Beispiel 10 verseift, so erhält man die oben verzeichnete Säure. Fp. 68-69°C (Cyclohexan).

**Patentansprüche**

1. Dioxybenzoletherderivate der allgemeinen Fomel

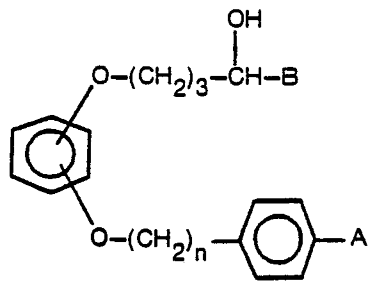

in welcher die beiden Ethergruppen in ortho- oder meta-Stellung zueinander stehen,
A eine der Gruppen
—COOH,
—$COOC_2H_5$,
—CH=CH—COOH oder
—CH=CH—$COOC_2H_5$,
B einen geradkettigen Alkylrest mit 3—6 C-Atomen und
n 1—3
bedeuten, sowie deren Additionssalze.

2. (E)-4-[3-(4-Hydroxyoctyloxy)-phenoxymethyl]-zimtsäure-ethylester.
3. (E)-4-[3-(4-Hydroxyoxyoctyloxy)-phenoxymethyl]-zimtsäure.
4. (E)-4-[3-(4-Hydroxynonyloxy)-phenoxymethyl]-zimtsäure-ethylester.
5. (E)-4-[3-(4-Hydroxynonyloxy-phenoxymethyl]-zimtsäure.
6. 4-3-[3-(4-Hydroxyheptyloxy)-phenoxy]-propyl-benzoesäureethylester.
7. 4-3-[3-(4-Hydroxyheptyloxy)-phenoxy]-propyl-benzoesäure.
8. (E)-4-[3-(4-Hydroxydecyloxy)-phenoxymethyl]-zimtsäureethylester.
9. (E)-4-[3-(4-Hydroxydecyloxy)-phenoxymethyl]-zimtsäure.
10. Dioxybenzoletherderivate der allgemeinen Formel

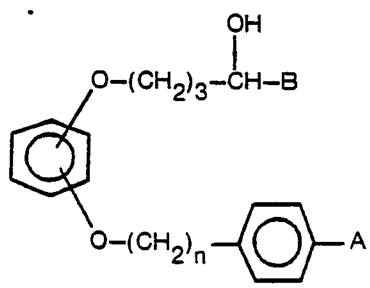

in welcher die beiden Ethergruppen in ortho- oder meta-Stellung zueinander stehen,
A eine der Gruppen
—COOH,
—$COOC_2H_5$,
—CH=CH—COOH oder
—CH=CH—$COOC_2H_5$,
B einen geradkettigen Alkylrest mit 3—6 C-Atomen und
n 1—3
bedeuten, sowie deren Additionssalze bei der Verwendung als Thromboxan-Synthetase-Hemmer.

11. Verwendung von Dioxybenzoletherderivaten der allgemeinen Formel

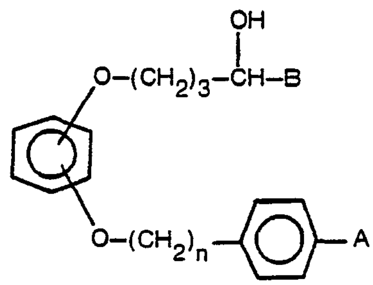

in welcher die beiden Ethergruppen in ortho- oder meta-Stellung zueinander stehen,
A eine der Gruppen
—COOH,
$—COOC_2H_5$,
—CH=CH—COOH oder
$—CH=CH—COOC_2H_5$,
B einen geradkettigen Alkylrest mit 3—6 C-Atomen und
n 1—3
bedeutet, sowie deren Additionssalzen zur Herstellung von Arzneimitteln mit Thromboxan-Synthetase-Hemmwirkung.

12. Verfahren zur Herstellung von Dioxybenzoletherderivate der allgemeinen Formel

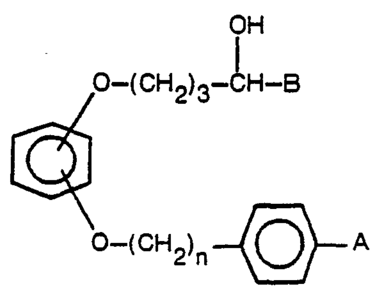

in welcher die beiden Ethergruppen in ortho- oder meta-Stellung zueinander stehen,
A eine der Gruppen
—COOH,
$—COOC_2H_5$,
—CH=CH—COOH oder
$—CH=CH—COOC_2H_5$,
B einen geradkettigen Alkylrest mit 3—6 C-Atomen und
n 1—3
bedeutet, sowie deren Additionssalzen,
dadurch gekennzeichnet, daß man Hydroxybenzolderivate der allgemeinen Formel

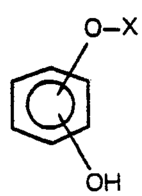

in welcher
X für eine Schutzgruppe steht,
in inerten organischen aprotischen Lösungsmitteln in Gegenwart der äquivalenten Menge einer starken Base mit Verbindungen der allgemeinen Formel

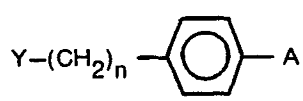

wobei n und A die oben angegebene Bedeutung haben und Y eine austretende Gruppe darstellt, bei Temperaturen zwischen 0 und 150°C umsetzt, anschließend die Schutzgruppe X nach üblichen

Methoden abspaltet und dann mit Verbindungen der allgemeinen Formel

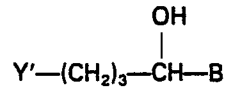

wobei
B die oben angegebene Bedeutung hat und
Y' die Bedeutung von Y besitzt, ohne mit dieser identisch sein zu müssen,
in der oben beschriebenen Weise zu Verbindungen der allgemeinen Formel in Anspruch 1 verethert.

13. Arzneimittel mit Thromboxan-Synthesetase-Hemmwirkung, enthaltend Dioxybenzoletherderivate der allgemeinen Formel

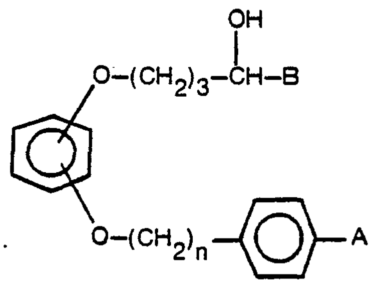

in welcher die beiden Ethergruppen in ortho- oder meta-Stellung zueinander stehen,
A eine der Gruppen
$-COOH$,
$-COOC_2H_5$,
$-CH=CH-COOH$ oder
$-CH=CH-COOC_2H_5$,
B einen geradkettigen Alkylrest mit 3—6 C-Atomen und
n 1—3
bedeutet und oder deren Additionssalze.

14. Verfahren zur Herstellung von Arzneimitteln, dadurch gekennzeichnet, daß man Dioxybenzoletherderivate gemäß den Ansprüchen 1, gegebenenfalls unter Zusatz von inerten pharmazeutisch unbedenklichen Hilfs- und Trägerstoffen in eine geeignete Applikationsform überführt.

**Revendications**

1. Dérivés d'éthers dioxybenzéniques de formule générale

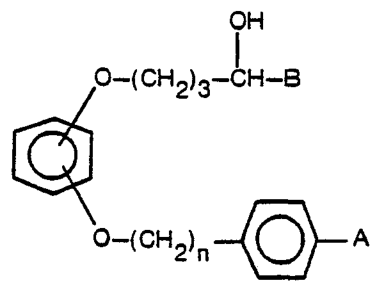

dans laquelle les deux groupes éther sont en position ortho ou méta l'un par rapport à l'autre,
A désigne l'un des groupes
$-COOH$,
$-COOC_2H_5$,
$-CH=CH-COOH$ ou
$-CH=CH-COOC_2H_5$,
B est un reste alkyle à chaîne droite ayant 3 à 6 atomes de carbone et
n a une valeur de 1 à 3,
ainsi que leurs sels d'addition d'acides.

2. L'ester éthylique de l'acide (E)-4-[3-(4-hydroxyoctyloxy)-phénoxyméthyl]-cinnamique.
3. L'acide (E)-4-[3-(4-hydroxyoxyoctyloxy)-phénoxyméthyl]cinnamique.
4. L'ester éthylique de l'acide (E)-4-[3-(4-hydroxynonyloxy)-phénoxyméthyl]-cinnamique.
5. L'acide (E)-4-[3-(4-hydroxynonyloxy-phénoxy-méthyl]-cinnamique.
6. L'ester éthylique de l'acide 4-3-[3-(4-hydroxyheptyloxy)-phénoxy]-propyl-benzoïque.
7. L'acide 4-3-[3-(4-hydroxyheptyloxy)-phénoxy]-propyl-benzoïque.
8. L'ester éthylique de l'acide (E)-4-[3-(4-hydroxydécyloxy)-phénoxyméthyl]-cinnamique.

9. L'acide (E)-4-[3-(4-hydroxydécyloxy)-phénoxyméthyl]-cinnamique.

10. Dérivés d'éthers dioxybenzéniques de formule générale

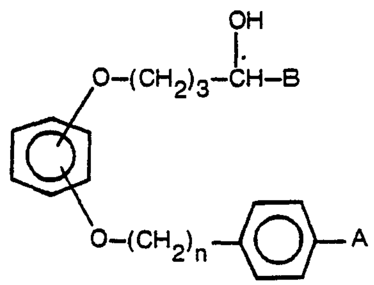

dans laquelle les deux groupes éther sont en position ortho ou en position méta l'un par rapport à l'autre,

A est l'un des groupes
$-COOH$,
$-COOC_2H_5$,
$-CH{=}CH-COOH$ ou
$-CH{=}CH-COOC_2H_5$,

B est un reste alkyle à chaîne droite ayant 3 à 6 atomes de carbone et

n a une valeur de 1 à 3,

ainsi que leurs sels d'addition lors de leur utilisation comme inhibiteurs de thromboxane-synthétase.

11. Utilisation de dérivés d'éthers dioxybenzéniques de formule générale

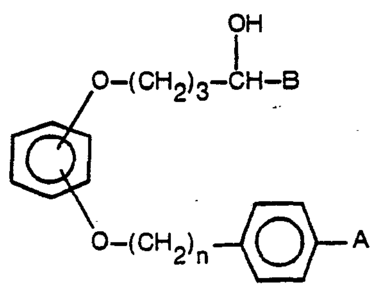

dans laquelle les deux groupes éther sont en position ortho ou en position méta l'un par rapport à l'autre,

A représente l'un des groupes
$-COOH$,
$-COOC_2H_5$,
$-CH{=}CH-COOH$ ou
$-CH{=}CH-COOC_2H_5$,

B est un reste alkyle à chaîne droite ayant 3 à 6 atomes de carbone et

n a une valeur de 1 à 3,

ainsi que de leur sels d'addition pour l'obtention de médicaments doués d'activité inhibitrice de thromboxane-synthétase.

12. Procédé de production de dérivés d'éthers dioxybenzéniques de formule générale

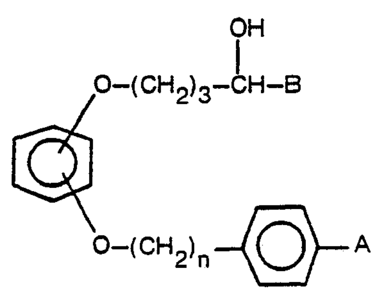

dans laquelle les deux groupes éther sont en position ortho ou méta l'un par rapport à l'autre,

A désigne l'un des groupes
$-COOH$,
$-COOC_2H_5$,
$-CH{=}CH-COOH$ ou
$-CH{=}CH-COOC_2H_5$,

B est un reste alkyle à chaîne droite ayant 3 à 6 atomes de carbone et

n a une valeur de 1 à 3,

ainsi que leurs sels d'addition, caractérisé en ce qu'on fait réagir des dérivés hydroxybenzéniques de formule générale

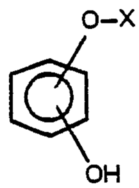

dans laquelle
X désigne un groupe protecteur,
dans des solvants organiques aprotiques inertes en présence de la quantité équivalente d'une base forte, avec des composés de formule générale

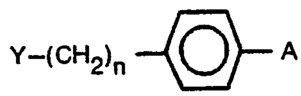

dans laquelle n et A ont la définition indiquée ci-dessus et Y désigne un groupe partant, à des températures comprises entre 0 et 150°C, puis on élimine le groupe protecteur X par des procédés classiques et on effectue ensuite une éthérification avec des composés de formule générale

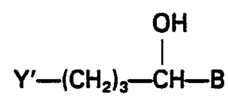

dans laquelle
B a la définition indiquée ci-dessus et
Y' a la définition de Y, sans devoir être identique à celui-ci,
de la manière décrite ci-dessus en composés de formule générale suivant la revendication 1.

13. Médicament doué d'activité inhibitrice de thromboxane-synthétase, contenant des dérivés d'éthers dioxybenzéniques de formule générale

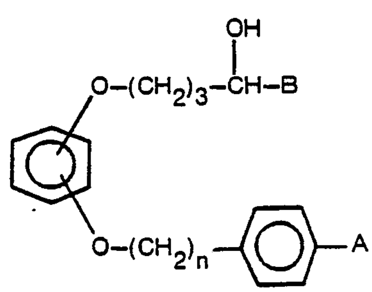

dans laquelle les deux groupes éther sont en position ortho ou méta l'un par rapport à l'autre,
A représente l'un des groupes
—COOH,
—$COOC_2H_5$,
—CH=CH—COOH ou
—CH=CH—$COOC_2H_5$,
B est un reste alkyle à chaîne droite ayant 3 à 6 atomes de carbone et
n a une valeur de 1 à 3
et/ou leurs sels d'addition.

14. Procédé de préparation de médicaments, caractérisé en ce qu'on transforme des dérivés d'éthers dioxybenzéniques suivant la revendication 1, éventuellement avec addition d'adjuvants et de supports inertes acceptables du point de vue pharmaceutique, en une forme d'application appropriée.

**Claims**

1. Dihydroxybenzene ether derivatives of the general formula

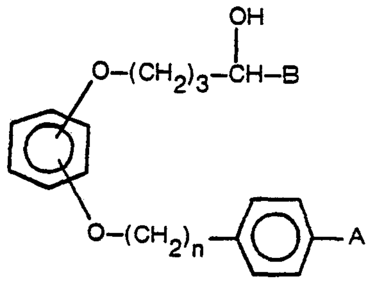

in which
the two ether groups are in the ortho or meta position to each other,
A denotes one of the groups
—COOH,
—$COOC_2H_5$,
—CH=CH—COOH or
—CH=CH—$COOC_2H_5$,
B denotes a straight-chain alkyl radical with 3—6 C atoms and
n denotes 1—3,
and addition salts thereof.

2. (E)-4-[3-(4-hydroxyoctyloxy)-phenoxymethyl]-cinnamic acid ethyl ester.
3. (E)-4-[3-(4-hydroxyoxyoctyloxy)-phenoxymethyl]-cinnamic acid.
4. (E)-4-[3-(4-hydroxynonyloxy)-phenoxymethyl]-cinnamic acid ethyl ester.
5. (E)-4-[3-(4-hydroxynonyloxy)-phenoxymethyl]-cinnamic acid.
6. 4-3-[3-(4-hydroxyheptyloxy)-phenoxy]-propyl-benzoic acid ethyl ester.
7. 4-3-[3-(4-hydroxyheptyloxy)-phenoxy]-propyl-benzoic acid.
8. (E)-4-[3-(4-hydroxydecyloxy)-phenoxymethyl]-cinnamic acid ethyl ester.
9. (E)-4-[3-(4-hydroxydecyloxy)-phenoxymethyl]-cinnamic acid.
10. Dihydroxybenzene ether derivatives of the general formula

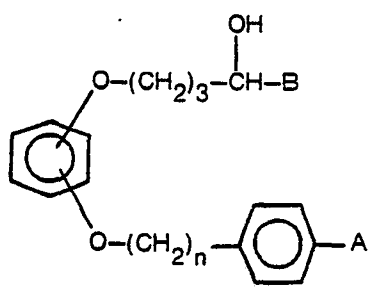

in which
the two ether groups are in the ortho or meta position to each other,
A denotes one of the groups
—COOH,
—$COOC_2H_5$,
—CH=CH—COOH or
—CH=CH—$COOC_2H_5$,
B denotes a straight-chain alkyl radical with 3—6 C atoms and
n denotes 1—3,
and addition salts thereof for use as inhibitors of thromboxan synthetase.

11. Use of dihydroxybenzene ether derivatives of the general formula

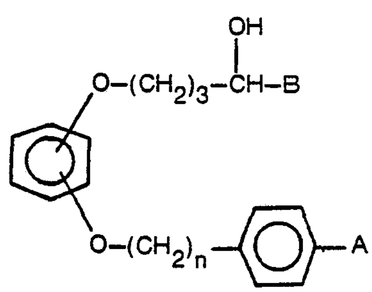

in which

the two ether groups are in the ortho or meta position to each other,

A denotes one of the groups

$-COOH$,

$-COOC_2H_5$,

$-CH=CH-COOH$ or

$-CH=CH-COOC_2H_5$,

B denotes a straight-chain alkyl radical with 3—6 C atoms and

n denotes 1—3,

and addition salts thereof for the preparation of medicaments having inhibitory action on thromboxan synthetase.

12. Process for the preparation of dihydroxybenzene ether derivatives of the general formula

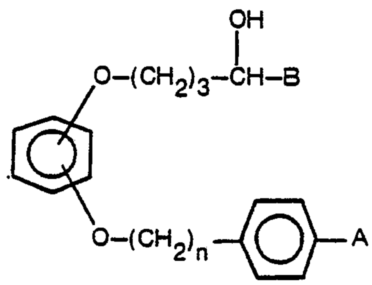

in which

the two ether groups are in the ortho or meta position to each other,

A denotes one of the groups

$-COOH$,

$-COOC_2H_5$,

$-CH=CH-COOH$ or

$-CH=CH-COOC_2H_5$,

B denotes a straight-chain alkyl radical with 3—6 C atoms and

n denotes 1—3,

and addition salts thereof, characterised in that hydroxybenzene derivatives of the general formula

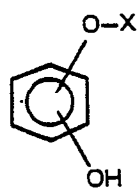

in which

X represents a protective group, are reacted in inert organic aprotic solvents in the presence of the equivalent quantity of a strong base with compounds of the general formula

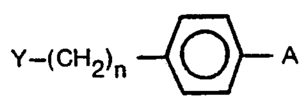

wherein

n and A have the meaning given above and

Y represents a leaving group,

at temperatures between 0 and 150°C, then the protective group X is split off by customary methods and the product is then etherified with compounds of the general formula

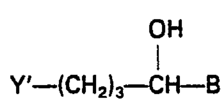

wherein

B has the meaning given above and

Y' has the meaning of Y, without having to be identical to the latter,

in the manner described above, to give compounds of the general formula in Claim 1.

13. Medicaments having inhibitory action on thromboxan synthetase, containing dihydroxybenzene ether derivatives of the general formula

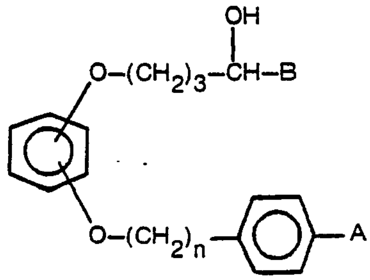

in which
the two ether groups are in the ortho or meta position to each other,
A denotes one of the groups
—COOH,
—$COOC_2H_5$,
—CH=CH—COOH or
—CH=CH—$COOC_2H_5$,
B denotes a straight-chain alkyl radical with 3—6 C atoms and
n denotes 1—3,
and/or addition salts thereof.

14. Process for the preparation of medicaments, characterised in that dihydroxybenzene ether derivatives according to Claims 1, are converted into a suitable form of administration, optionally with the addition of inert pharmaceutically harmless auxiliaries and excipients.